## Europäisches Patentamt
## European Patent Office
## Office européen des brevets

(19)

(11) Veröffentlichungsnummer: **0 000 341**
**B1**

(12) **EUROPÄISCHE PATENTSCHRIFT**

(21) Anmeldenummer: **78100217.5**

(51) Int. Cl.³: **C 07 C 97/26, C 07 C 79/37**

(22) Anmeldetag: **22.06.78**

(54) **Verfahren zur Herstellung von praktisch reinem 1-Amino-8-nitro-4,5-dihydroxy-anthrachinon.**

(30) Priorität: **07.07.77 DE 2730720**

(43) Veröffentlichungstag der Anmeldung:
**24.01.79 Patentblatt 79/2**

(45) Bekanntmachung des Hinweises auf die Patenterteilung:
**25.02.81 Patentblatt 81/8**

(84) Benannte Vertragsstaaten:
**BE CH DE FR GB**

(56) Entgegenhaltungen:
**DE - A - 1 929 808**
**DE - A - 2 024 780**
**FR - A - 2 260 563**

**DERWENT JAPANESE PATENTS REPORT,**
**Vol. 8, Nr. 37,2, Seite 2, "Abstract" 21432**

(73) Patentinhaber: **BASF Aktiengesellschaft**
**Carl-Bosch-Strasse 38**
**D-6700 Ludwigshafen (DE)**

(72) Erfinder: **Epple, Gerhard, Dr.**
**Karl-Gayer-Strasse 8**
**D-6719 Weisenheim (DE)**
(72) Erfinder: **Elser, Wolfgang, Dr.**
**Theodor-Heuss-Strasse 4**
**D-6706 Wachenheim (DE)**
(72) Erfinder: **Himmele, Walter, Dr.**
**Eichenweg 14**
**D-6909 Walldorf (DE)**

EP 0 000 341 B1

Courier Press, Leamington Spa, England.

Verfahren zur Herstellung von praktisch reinem 1-Amino-8-nitro-4,5-dihydroxy-anthrachinon

Die vorliegende Erfindung betrifft ein Verfahren zur Herstellung von praktisch reinem 1 - Amino - 8 - nitro - 4,5 - dihydroxy - anthrachinon durch partielle Reduktion von 1,8 - Dinitro - 4,5 - dihydroxyanthrachinon.

Aus der CH—PS 370 857 ist bekannt, daß sich Dinitro - dihydroxyanthrachinone im alkalisch wäßrigen Medium mit Natriumhydrogensulfid oder mit reduzierenden Zuckern, wie Glukose reduzieren lassen. In den DE—OS 24 28 338 und 24 28 452 wird die partielle Reduktion von Dinitro - dihydroxyanthrachinon mit Hydrazin in wäßrigem Medium bzw. in organischen protischen Lösungsmitteln beschrieben.

Ein für die Herstellung von wertvollen, z.B. von in der DE—OS 20 29 793 beschriebenen Blaufarbstoffen geeignetes 1 - Amino - 8 - nitro - 4,5 - dihydroxy - anthrachinon erhält man nach den Verfahren des Standes der Technik nur, wenn reines, von $\beta$ - Nitroverbindungen freies 1,8 - Dinitro - 4,5 - dihydroxyanthrachinon als Ausgangsprodukt verwendet wird. Letzteres kann nach dem Stand der Technik nur auf folgendem Wege erhalten werden: 4,5-Dichlor- oder 4,5 - Dinitro - anthrachinon wird mit Phenol zum 4,5 - Diphenoxyanthrachinon umgesetzt. Durch Nitrierung dieser Verbindung erhält man ein 4,5 - Bis - (2',4' - dinitro - phenoxy) - 1,8 - dinitro - anthrachinon, das durch alkalische Spaltung in das Dinatriumsalz des 4,5 - Dihydroxy - 1,8 - dinitro - anthrachinon und schließlich durch Ansäuern in das 1,8 - Dinitro - 4,5 - dihydroxyanthrachinon — im folgenden auch Dinitrochrysazin genannt — überführt wird (Endeavour *35*, Seite 137 (September 1976)).

Der vorliegenden Erfindung lag die Aufgabe zugrunde, ein technisch sicher zu handhabendes Verfahren aufzufinden, nach dem ein für Farbstoffsynthesen geeignetes, d.h. ein praktisch reines 1 - Amino - 8 - nitro - 4,5 - dihydroxy - anthrachinon aus dem durch direkte Nitrierung von 4,5 - Dihydroxy - anthrachinon (= Chrysazin) erhältliches rohes Dinitrochrysazin hergestellt werden kann.

Es wurde gefunden, daß man praktisch reines 1 - Amino - 8 - nitro - 4,5 - dihydroxy - anthrachinon durch partielle Reduktion von 1,8 - Dinitro - 4,5 - dihydroxyanthrachinon erhalten kann, wenn man durch Nitrierung von 4,5 - Dihydroxyanthrachinon erhaltenes rohes 1,8 - Dinitro - 4,5 - dihydroxyanthrachinon in einem Phenol - Wasser - Gemisch, das 5 bis 50 Gewichtsprozent Wasser enthält, in Gegenwart von Alkalimetallphenolat mit Hydroxyaceton, Dihydroxyaceton oder Gemischen davon partiell reduziert.

Nach dem erfindungsgemäßen Verfahren erhält man aus dem durch Nitrierung von 4,5 - Dihydroxyanthrachinon erhältlichen rohen 1,8 - Dinitro - 4,5 - dihydroxyanthrachinon ein praktisch chromatographisch einheitliches 1 - Amino - 8 - nitro - 4,5 - dihydroxy - anthrachinon, das für die Herstellung von Farbstoffen hervorragend geeignet ist. Überraschend war, daß bei dem erfindungsgemäßen Verfahren die bei der Nitrierung entstandenen Neben-, produkte und/oder die daraus entstehenden Reduktionsprodukte selektiv und praktisch vollständig vom gewünschten 1 - Amino - 8 - nitro - 4,5 - dihydroxyanthrachinon abgetrennt werden.

Der wirtschaftliche Vorteil des erfindungsgemäßen Verfahrens liegt darin, daß anstelle des über eine aufwendige und über mehrere Stufen verlaufende Synthese erhältlichen reinen Dinitrochrysazins, das durch direkte Nitrierung von Chrysazin erhältliche rohe Dinitrochrysazin zur partiellen Reduktion verwendet werden kann.

Das erfindungsgemäße Verfahren wird im allgemeinen so durchgeführt, daß man das rohe Dinitrochrysazin in Phenol und Wasser suspendiert. Das Alkalimetallphenolat wird dann entweder als solches zugegeben oder vorteilhafterweise durch Zugabe von Alkalihydroxid oder -karbonat im Reaktionsgemisch erzeugt. Das Reduktionsmittel wird anschließend bei Temperaturen zwischen 20 und 100°C unter Rühren zugegeben und das Reaktionsgemisch bei der gewünschten Temperatur gehalten, bis kein Dinitrochrysazin mehr vorhanden ist. Nach dem Abkühlen wird das Reaktionsgemisch filtriert, der Filterkuchen mit Methanol und/oder mit verdünnter Sodalösung phenolfrei gewaschen, gegebenenfalls mit Wasser neutral gewaschen und getrocknet.

Als Reaktionsmedium dient ein Gemisch aus Phenol und Wasser. Der Wassergehalt liegt zwischen 5 und 50 Gewichtsprozent, vorzugsweise zwischen 30 und 45 Gewichtsprozent, bezogen auf das Gemisch. Vorteilhafterweise wird die Suspension des rohen Dinitrochrysazins dadurch erhalten, daß man das rohe Dinitrochrysazin in Form des wäßrigen Preßkuchens zum vorgelegten Phenol gibt. Gegebenenfalls wird Phenol nachgesetzt, damit der Wassergehalt in der Phenol-/Wassermischung unterhalb von 50 Gewichtsprozent liegt oder Wasser als azeotropes Gemisch mit Phenol oder anderen Schleppmitteln abdestilliert.

Die Menge an Reaktionsmedium liegt im allgemeinen zwischen dem 2- und 10-, vorzugsweise zwischen dem 3- und 6-fachen, bezogen auf angewandtes rohes Dintrochrysazin. Die Menge an Reaktionsmedium ist einerseits vom Phenolgehalt, andererseits vom Gehalt des rohen Dinitrochrysazins an Nebenprodukten abhängig. Bei höherem Phenolgehalt kann die Menge an Reaktionsmedium geringer sein, während höhere Gehalte an Verunreinigungen eine um 10 bis 25% größere Menge an Reaktionsmedium bedingen.

In der Regel wendet man je Mol Dinitro - dihydroxyanthrachinon 0,2 bis ungefähr 1,5 Mol an Phenolat an, wobei die Menge vom Wassergehalt des Reaktionsmediums, von Reduktionsmittel und von dessen angewendeter Menge abhängig ist.

Die zur partiellen Reduktion erforderliche Menge an Reduktionsmittel hängt vom Reduktionsmittel selbst und von den Reduktionsbedingungen ab.

Die erforderliche Menge an Reduktionsmittel wird zweckmäßigerweise durch Versuche unter den später anzuwendenden Reaktionsbedingungen ermittelt. Hierbei werden Proben des Reaktionsproduktes chromatographisch untersucht und festgestellt, welche Menge Reduktionsmittel erforderlich ist, bis alles Dinitrochrysazin verschwunden ist.

Aus wirtschaftlichen Gründen kann als Reduktionsmittel technisches Hydroxyaceton verwendet werden, das noch Propylenglykol enthält.

Die Reduktion erfolgt im allgemeinen bei Temperaturen zwischen Raumtemperatur (20°C) und 100°C, vorzugsweise zwischen 80 und 100°C, insbesondere bei der Siedetemperatur des Phenol - Wasser - Gemisches.

Je nach den Bedingungen ist die Reduktion in der Regel in 0,5 bis 3 Stunden beendet. Die Reaktion ist beendet, wenn in einer Probe kein Dinitrochrysazin mehr festgestellt werden kann; gegebenenfalls wird dem Reaktionsgemisch noch Reduktionsmittel und — falls erforderlich — auch Alkalihydroxid zur Bildung von Phenolat nachgegeben.

Nach dem Verfahren erhält man aus rohem Dinitrochrysazin, das durch direkte Nitrierung von Chrysazin erhalten wird, ein praktisch reines 1 - Amino - 8 - nitro - 4,5 - dihydroxyanthrachinon. Das Verfahrensprodukt ist für alle Verwendungszwecke geeignet.

Die folgenden Ausführungsbeispiele sollen das erfindungsgemäße Verfahren weiter erläutern. Die genannten Teile und Prozentangaben beziehen sich auf das Gewicht.

### Beispiel 1

498 Teile wäßriger Preßkuchen des rohen Dinitrochrysazins (Gehalt: 33, 1 %, ≙ 165 Teile trocken), 550 Teile Phenol und 32,5 Teile 50 %ige Natronlauge werden gemischt und auf 90 bis 95°C erhitzt. Unter Rühren werden innerhalb von 0,5 Stunden 100 Teile Hydroxyaceton roh (78 %ig) zugetropft. Anschließend wird das Reaktionsgemisch 1,5 Stunden unter Rückfluß gekocht. Nach dem Abkühlen wird das ausgefallene Reaktionsprodukt abgesaugt, das Filtergut mit 1 000 Teilen 2 %iger Natriumcarbonatlösung und heißem Wasser nachgewaschen und getrocknet. Man erhält 109 Teile dünnschichtchromatographisch einheitliches 1 - Amino - 8 - nitro - 4,5 - dihydroxyanthrachinon.

Das als Ausgangsprodukt verwendete rohe Dinitrochrysazin wurde wie folgt hergestellt:

4 000 Teile 23 %iges Oleum und 248 Teile Borsäure werden unter Rühren 1 Stunde auf 50°C erwärmt. Nach dem abkühlen auf 30°C werden 360 Teile Chrysazin ber. 100 % (≙ 400 Teile 90 %ig) so eingetragen, daß die Temperatur nicht über 50°C steigt. Anschließend erwärmt man 2 Stunden auf 50°C, kühlt auf 0°C ab und tropft unter Solekühlung bei 0 bis 5°C innerhalb von 3 Stunden 528 kg Mischsäure (48 % $HNO_3$) zu. Nach beendetem Zutropfen wird auf Eiswasser ausgetragen. Das ausgefallene Reaktionsprodukt wird abgesaugt und mit Wasser neutral gewaschen. Ausbeute: 528 Teile rohes Dinitrochrysazin berechnet als trockenes Produkt in Form des wäßrigen Preßkuchens (Feststoffgehalt: 33,1 %).

### Beispiel 2

472 Teile wäßriger Preßkuchen eines rohen Dinitrochrysazins (Gehalt 35,2 %ig = 165 Teile trocken), 550 Teile Phenol und 40 Teile 50 %ige Natronlauge werden unter Rühren auf 95 bis 100°C erwärmt. Bei dieser Temperatur gibt man innerhalb von 0,5 Stunden 111 Teile Hydroxyaceton (ca. 98 %ig) zu und erhitzt noch 1 Stunde unter Rückfluß. Nach dem Abkühlen saugt man das ausgefallene Reaktionsprodukt ab, wäscht mit 500 Teilen 2 %iger Sodalösung und heißem Wasser. Nach dem Trocknen erhält man 111 Teile 1 - Amino - 8 - nitro - 4,5 - dihydroxy - anthrachinon, das noch geringe Mengen an Nebenprodukten enthält.

### Beispiel 3

50 Teile wäßriger Preßkuchen eines rohen Dinitrochrysazins (Gehalt: 33,1 %ig = 16,5 Teile trocken), 60 Teile Phenol und 3,24 Teile 50 %ige natronlauge werden auf 90 bis 100°C erhitzt. Innerhalb von 0,5 Stunden werden unter Rühren 10 Teile Dihydroxyaceton eingetragen. Zur Vervollständigung der Reaktion wird noch 1,5 Stunden unter Rückfluß gekocht, dann abgekühlt und das ausgefallene Reaktionsprodukt abgesaugt. Man arbeitet wie in Beispiel 1 angegeben auf. Nach dem Trocknen erhält man 9,8 Teile chromatographisch einheitliches 1 - Amino - 8 - nitro - 4,5 - dihydroxyanthrachinon.

### Beispiel 4

50 Teile wäßriger Preßkuchen des rohen Dinitrochrysazins (Gehalt 33,1 %ig ≙ 16,5 Teile trocken), 60 Teile Phenol und 5,5 Teile wasserfreies Kaliumcarbonat werden auf 90 bis 95°C erhitzt. Unter Rühren werden innerhalb von 0,5 Stunden 10 Teile Hydroxyaceton zugetropft. Das Reaktionsgemisch wird 3 Stunden auf Rückflußtemperatur erhitzt, anschließend abgekühlt und das ausgefallene Reaktionsprodukt abgesaugt. Nach dem Waschen mit 500 Teilen 2 %iger Sodalösung und heißem Wasser wird das Filtergut getrocknet. Man erhält 11,0 Teile 1 - Amino - 8 - nitro - 4,5 - dihydroxy - anthrachinon, das nur noch Spuren an Verunreinigungen enthält.

### Beispiel 5

Man verfährt wie in Beispiel 4 angegeben. Anstelle von 5,5 Teilen Kaliumcarbonat werden jedoch 4,25 Teile wasserfreies Natriumcarbonat verwendet. Man erhält 10,3 Teile 1 - Amino - 8 - nitro - 4,5 - dihydroxy - anthrachinon, das chromatographisch praktisch einheitlich ist.

### Patentanspruch

1. Verfahren zur Herstellung von praktisch reinem 1 - Amino - 8 - nitro - 4,5 - dihydroxyanthrachinon partielle Reduktion von 1,8 - Dinitro - 4,5 - dihydroxyanthrachinon in wäßrigem Medium und Isolierung des Amino - nitro - dihydroxyanthrachinons, *dadurch gekennzeichnet*, daß man rohes 1,8 - Dinitro - 4,5 - dihydroxyanthrachinon, das durch Nitrierung von 4,5 - Dihydroxyanthrachinon erhalten wird, in einem Phenol - Wasser - Gemisch, das 5 bis 50 Gewichtsprozent Wasser enthält, in Gegenwart von Alkalimetallphenolat mit Hydroxyaceton, Dihydroxyaceton oder Gemischen davon partiell reduziert.

### Claim

1. A process for the production of virtually pure 1 - amino - 8 - nitro - 4,5 - dihydroxyanthraquinone by partially reducing 1,8 - dinitro - 4,5 - dihydroxyanthraquinone in aqueous medium and isolating the amino - nitro - dihydroxyanthraquinone, *characterised in that* crude 1,8 - dinitro - 4,5 - dihydroxyanthraquinone obtained by nitration of 4,5 - dihydroxyanthraquinone is partially reduced in a phenol-water mixture, which contains from 5 to 50% by weight of water, in the presence of an alkali metal phenolate, by means of hydroxyacetone, dihydroxyacetone or a mixture of these.

### Revendication

1. Procédé pour la préparation de 1 - amino - 8 - nitro - 4,5 - dihydroxyanthraquinone pratiquement pure par réduction partielle de 1,8 - dinitro - 4,5 - dihydroxyanthraquinone en milieu aqueux et isolement de l'amino - nitro - dihydroxyanthraquinone, caractérisé en ce qu'on réduit partiellement une 1,8 - dinitro - 4,5 - dihydroxyanthraquinone brute, qui est obtenue par nitration de 4,5 - dihydroxyanthraquinone, dans un mélange de phénol et d'eau qui contient 5 à 50% en poids d'eau, en présence d'un phénolate de métal alcalin, avec un hydroxyacétone, une dihydroxyacétone ou des mélanges de ces acétones.